# EUROPEAN PATENT APPLICATION

(11) **EP 3 228 301 A1**
(43) Date of publication of application: **11.10.2017**
(21) Application number: 15865370.9
(22) Date of filing: 17.11.2015
(51) Int. Cl.: A61K 8/365, A61Q 5/04

(54) **HAIR SHAPING AGENT**

(30) Priority: 05.12.2014 JP 2014247486
(71) Applicant: Kao Corporation, Chuo-Ku Tokyo 103-8210 (JP)
(72) Inventor: FURUKAWA, Junichi, Tokyo 131-8501 (JP); TOKUNAGA, Shinichi, Tokyo 131-8501 (JP)
(74) Representative: Hoffmann Eitle
(86) International application number: PCT/JP2015/082285
(87) International publication number: WO 2016/088552

(57) **Abstract**

An agent for hair deforming treatment comprising the following components (A) and (B):
(A) a compound represented by the following formula (1), and
(B) water: 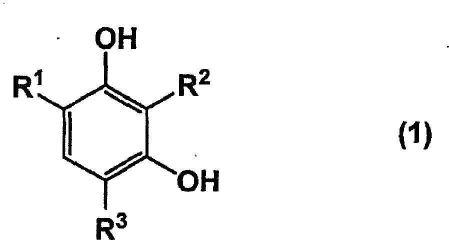 wherein R¹, R² and R³ represent H or a group represented by formula (2), and 0 to 2 thereof are H, wherein,
in the formula (2), R represents H or a carboxy group, and A¹ and A² represent H, OH or a group represented by formula (3), wherein
in the formula (3), any one of X, Y and X represents a site binding to the C in the formula (2), and the others represent H or -CH(OH)COOH.

## Description

### [Field of the Invention]

The present invention relates to an agent for hair deforming treatment capable of semi-permanently or permanently deforming the shape of hair.

### [Background of the Invention]

As methods for semi-permanently or permanently deforming the shape of hair, there have been a method of using a reducing agent, as in the case of the formation of permanent wave, and a method of using a strongly-alkaline treatment agent having pH 12 to 14, including an alkali relaxer as a representative example. However, it has been well known that these methods pose a great burden on hair, and thus that damage has easily remained on the hair. In addition, in recent years, as a method for semi-permanently or permanently the shape of hair with less damage to the hair, a hair-relaxing method, in which a large amount of formaldehyde is used, has been developed. However, such a method of using highly toxic formaldehyde must be handled and operated carefully, because of its high volatility, and thus, it cannot be said that this is a preferred method for hair treatment.

Hence, a hair-relaxing method, which does not give damage to hair and does not use formaldehyde, and which is safer to a human body, has been searched. For example, Patent Literature 1 discloses a technique of applying α-keto acid, and particularly, glyoxylic acid to hair, and then subjecting the hair to a heat treatment with a flat iron at 200°C ± 50°C, to convert strongly curly hair to straight hair. Moreover, Patent Literature 2 discloses a method which comprises applying a polyhydroxylated aromatic compound to hair, and then heating the hair to a temperature of 110°C or higher, to permanently relax keratin fibers.

With regard to temporary hair deformation, such as the resetting of a hair style by rinsing the hair with water, it can be realized by using a styling agent. For example, Patent Literature 3 discloses a hair composition, which is formed by heating glyceraldehyde and resorcin to reflux in the presence of boric acid and silicic acid, to form a low-order condensate. Patent Literature 3 describes that this composition improves set retentivity and humidity resistance, is capable of reforming the hair style by wetting the hair with water, and improves the mechanical strength of hair.

(Patent Literature 1) EP 2538916 A
(Patent Literature 2) JP-A-2009-537619
(Patent Literature 3) US 4278659 B

### [Summary of the Invention]

The present invention provides an agent for hair deforming treatment comprising the following components (A) and (B):

### (A) a compound represented by the following formula (1):

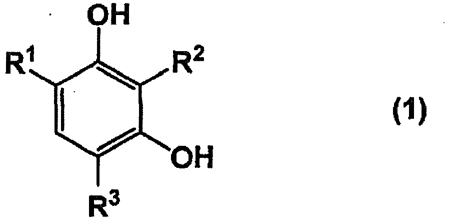

wherein R¹, R² and R³ each independently represent a hydrogen atom or a group represented by the following formula (2), wherein not all of R¹, R² and R³ simultaneously represent hydrogen atoms: 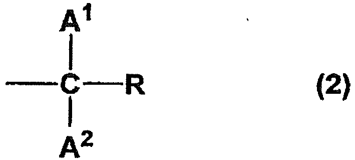 wherein R represents a hydrogen atom or a carboxy group, and A¹ and A² each independently represent a hydrogen atom, a hydroxyl group or a group represented by the following formula (3): 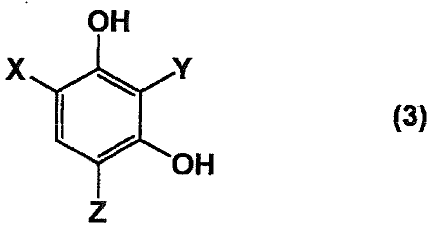 wherein any one of X, Y and Z represents a bond binding to the carbon atom shown in the formula (2), and the others represent a hydrogen atom or -CH(OH)COOH;

### (B) water.

Moreover, the present invention provides a method for hair treatment for semi-permanently or permanently deforming the shape of hair, which comprises the following steps (i) and (ii):
(i) a step of applying the above described agent for hair deforming treatment to hair, and then allowing the agent to penetrate into the hair, and
(ii) a step of heating and shaping the hair into which the agent for hair deforming treatment has penetrated.

### [Detailed Description of the Invention]

When the hair treatment agent used in the method described in Patent Literature 1 or 2 is applied to hair, even if it could convert the hair to straight hair semi-permanently, it could not give a semi-permanently or permanently wavy or curly shape to the hair. In addition, when the once formed semi-permanent or permanent straight shape intends to be converted to another semi-permanent or permanent hair shape such as a wavy or curly shape, it is necessary to perform again the conventional operation of using a reducing agent. Thus, enormous amounts of time and efforts are required, and further, hair must be damaged.

Meanwhile, in the technique described in Patent Literature 3, the surface of hair is treated with a low-order condensate obtained by heating a composition comprising glyceraldehyde and resorcin under reflux. This low-order condensate is a substance having a relatively large molecular weight, and the invention described in Patent Literature 3 is characterized in that a treatment agent can be washed away with water because such a low-order condensate is used as the treatment agent. Accordingly, by this technique, since the deformed hair shape is returned to the original hair shape by washing hair repeatedly, it is hardly said that this is semi-permanent or permanent hair shape deformation.

Accordingly, the present invention relates to An agent for hair deforming treatment and a method for hair treatment, which have less damage to hair, are able to semi-permanently or permanently give not only the shape of straight hair, but also a wavy or curly shape, when the agent and the method is applied to hair, and further, are able to easily convert the once formed hair shape to another hair shape semi-permanently or permanently, without using agent for hair deforming treatments such as a reducing agent, and without giving damage to the hair.

The present inventors have found that a hair treatment agent comprising a particular compound cannot only semi-permanently give a straight hair shape or a curly or wavy hair shape to hair, but it can also deform the hair that has once been treated with this hair treatment agent to a completely different, any shape, by only using a heating means such as a hair iron or a curler, without treating the hair with a hair treatment agent such as a reducing agent, thereby completing the present invention.

The agent for hair deforming treatment of the present invention is highly safe to human bodies, has less damage to hair, and can deform the shape of hair semi-permanently or permanently, and the hair deformed with the present agent for hair deforming treatment cannot be lost even if the hair is washed with a shampoo or the like. In addition, once the present agent for hair deforming treatment is applied to hair, it is not necessary to allow a hair treatment agent to penetrate into the hair again, and the hair can be freely and repeatedly deformed only by giving heat to the hair. Moreover, the hair, which has been repeatedly deformed by being heated, still has high hair washing resistance of the shape, and thus the shape of the hair is not lost by shampoo, water, etc.

In the present invention, "semi-permanent or permanent hair deformation" means that hair has extremely excellent hair washing resistance, and that the shape of hair is maintained, even if shampooing is repeatedly carried out thereon. Specifically, it means that, when the deformed hair is washed with shampoo, and the shampoo is then fully washed away with water, and the hair is then naturally dried, the shape of hair is maintained before and after the shampooing. It is to be noted that the expression "the shape of hair is maintained" means that, for example, in the case of wavy hair, the number of waves is not substantially different before and after shampooing, and in the case of straight hair, wavy or curly hair is not substantially generated as a result of shampooing.

In the present invention, "hair deformation" means deformation of hair, which is not caused by the cleavage and recombination of the S-S bond of proteins in hair, and it includes deformation of straight hair into curly hair or the like, and also, deformation of hair which has been deformed into wavy or curly hair, or naturally curly hair, to straight hair.

### [Component (A): compound represented by formula (1)]

The component (A) is a compound represented by the following formula (1): 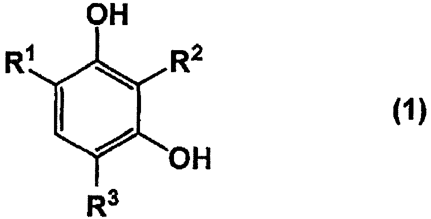 wherein R¹, R² and R³ each independently represent a hydrogen atom or a group represented by the following formula (2), wherein not all of R¹, R² and R³ simultaneously represent hydrogen atoms: 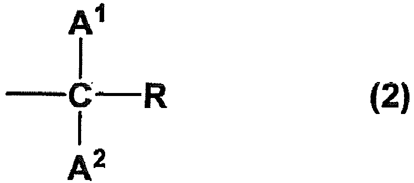 wherein R represents a hydrogen atom or a carboxy group, and A¹ and A² each independently represent a hydrogen atom, a hydroxyl group or a group represented by the following formula (3): 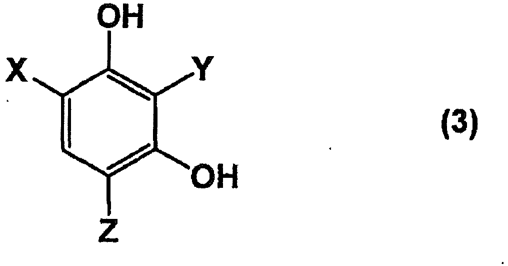 wherein any one of X, Y and Z represents a bond binding to the carbon atom shown in the formula (2), and the others represent a hydrogen atom or -CH(OH)COOH.

An example of the group represented by the formula (3) is a group, in which Y is a hydrogen atom, and either X or Z binds to the carbon atom shown in the formula (2) and the other is a hydrogen atom or -CH(OH)COOH.

Examples of the compound represented by the formula (1) include compounds having the following structural formulae:

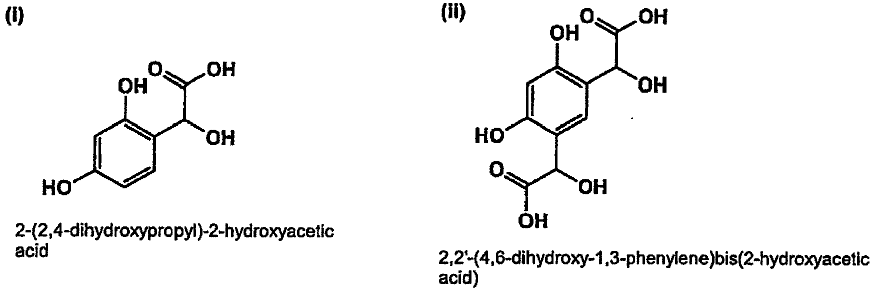

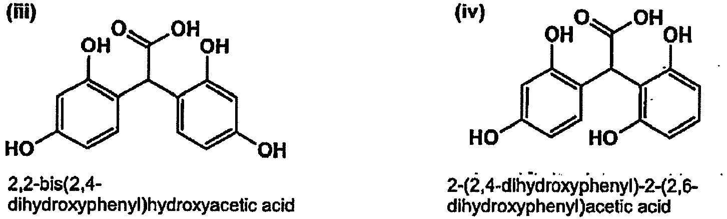

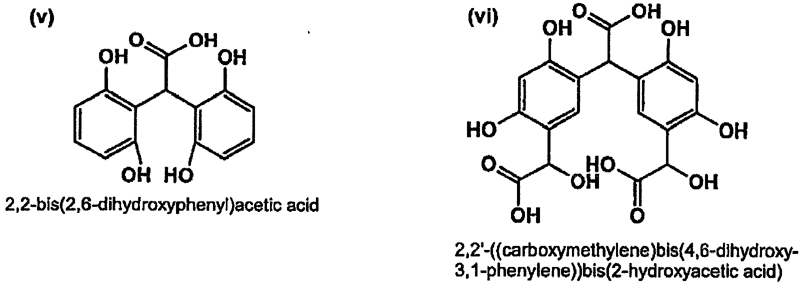

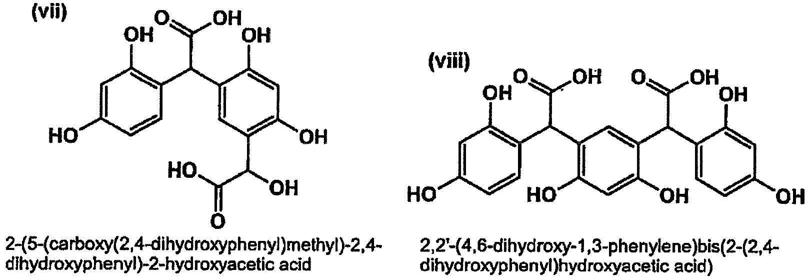

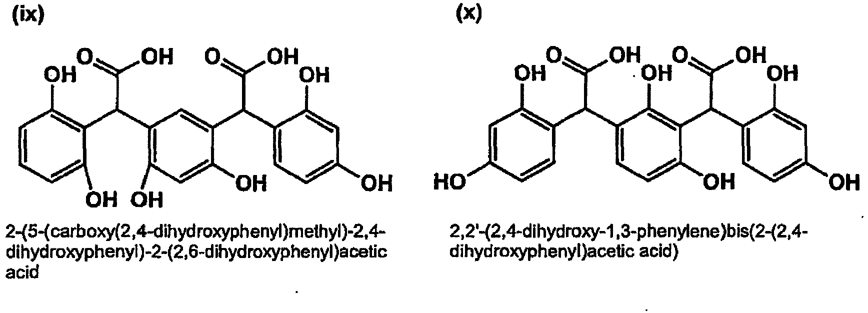

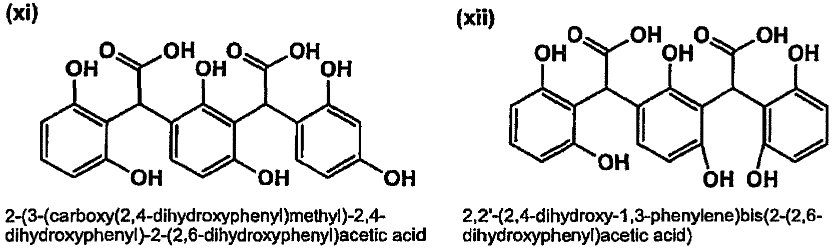

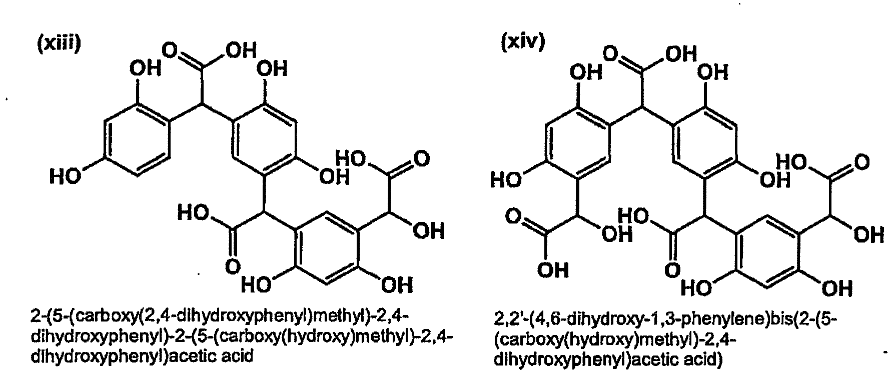

Moreover, The molecular weight of the compound represented by the formula (1) is preferably the molecular weight of 150 or more. In addition, from the viewpoint of permeability into hair, it is preferably the molecular weight of 1000 or less, more preferably 700 or less, and even more preferably 500 or less.

As such a component (A), a commercially available product may be used, or the component (A) may also be synthesized, for example, by reacting glyoxylic acid with resorcin.

The component (A) can be used alone or in combination of two or more components. From the viewpoint of achieving a more significant change in the shape of hair after the treatment of hair with the hair treatment agent of the present invention, achieving more excellent hair washing resistance of the shape of hair, achieving a more significant change in the shape of hair upon semi-permanent re-deformation of the shape of hair by heating, and also achieving more excellent hair washing resistance of the shape of hair after completion of the re-deformation, the content of the component (A) is preferably 0.1 mass% or more, more preferably 0.5 mass% or more, even more preferably 1 mass% or more, and further preferably 1.5 mass% or more. From the viewpoint of formulation mixing properties, it is preferably 30 mass% or less, more preferably 25 mass% or less, even more preferably 23 mass% or less, and further preferably 20 mass% or less.

### [Component (B): water]

The agent for hair deforming treatment of the present invention uses water as a medium. Moreover, in addition to water, lower alcohol containing from 1 to 3 carbon atoms, such as methanol or ethanol, can be used in combination with water, as necessary. In this case, the content of the lower alcohol containing from 1 to 3 carbon atoms in the agent for hair deforming treatment of the present invention is preferably 60 mass% or less, more preferably 40 mass% or less, even more preferably 30 mass% or less, further preferably 20 mass% or less, still further preferably 15 mass% or less, and still further preferably 10 mass% or less. In addition, it is preferably 0.1 mass% or more.

From the viewpoint of the permeability into hair, the pH of the agent for hair deforming treatment of the present invention is preferably 4 or less, more preferably 3 or less, even more preferably 2.5 or less, and further preferably 2 or less. In addition, from the viewpoint of suppression of hair damage and suppression of irritation to the skin, the pH of the present agent for hair deforming treatment is preferably 1 or more, more preferably 1.2 or more, and even more preferably 1.5 or more. In the present application, the pH of the hair treatment agent indicates a value obtained by directly measuring the pH of the agent for hair deforming treatment at a room temperature (25°C) using a pH meter, without dilution and the like of the agent for hair deforming treatment.

In order to adjust the pH of the agent for hair deforming treatment to be in the above described range, a pH adjuster can be used, as appropriate. Examples of the pH adjuster as an alkali agent that can be used include: ammonia or a salt thereof; alkanolamine such as monoethanolamine, isopropanolamine, 2-amino-2-methylpropanol or 2-aminobutanol, or a salt thereof; alkanediamine such as 1,3-propanediamine, or a salt thereof; carbonates such as guanidine carbonate, sodium carbonate, potassium carbonate, sodium hydrogen carbonate, or potassium hydrogen carbonate; and hydroxides such as sodium hydroxide or potassium hydroxide. Moreover, as an acid agent, inorganic acids such as hydrochloric acid or phosphoric acid, hydrochlorides such as monoethanolamine hydrochloride, phosphates, etc., such as monopotassium dihydrogen phosphate or disodium monohydrogen phosphate, or organic acids such as lactic acid or malic acid, can be used.

The agent for hair deforming treatment of the present invention may comprise, as appropriate, components generally mixed into hair cosmetics, as well as the aforementioned components. However, preferably, the agent for hair deforming treatment of the present invention substantially does not comprise a hair reducing agent. In particular, the agent for hair deforming treatment of the present invention substantially does not comprise a reducing agent selected from the group consisting of thiol, hydrogen sulfite, and a salt thereof, which are frequently comprised in conventional perm agents. The present invention is characterized in that it makes possible to deform hair, without the cleavage of the S-S bond of proteins in hair. Thus, the present invention is a technique completely different from a perm agent for deforming hair by cleaving the S-S bond of proteins in hair, using a reducing agent. Moreover, Examples of the aforementioned hair reducing agent include thioglycolic acid, dithioglycolic acid, cysteine, acetylcysteine, thiol such as butyrolactonethiol, hydrogen sulfite, and a salt thereof.

In the present invention, the expression "substantially does not comprise" is used to mean that the content of a target compound in the agent for hair deforming treatment is preferably less than 0.1 mass%, more preferably less than 0.01 mass%, and even more preferably, it means that the agent for hair deforming treatment does not comprise a target compound.

### [Method for hair deforming treatment]

A hair treatment for semi-permanently or permanently deforming the shape of hair using the agent for hair deforming treatment of the present invention can be carried out by a method for hair treatment comprising the following steps (i) and (ii):
(i) a step of applying the agent for hair deforming treatment of the present invention to hair, and then allowing the agent to penetrate into the hair, and
(ii) a step of heating and shaping the hair into which the agent for hair deforming treatment has penetrated.

In the step (i), the agent for hair deforming treatment may be applied to dry hair or may also be applied to wet hair. In order to swell hair and to promote penetration of the hair treatment agent into the hair, hair is preferably wetted with water before the step (i). The mass of the agent for hair deforming treatment applied to hair in the step (i) is, at a bath ratio to the mass of the hair (the mass of the agent for hair deforming treatment / the mass of the hair), preferably 0.05 or more, more preferably 0.1 or more, even more preferably 0.25 or more, and further preferably 0.5 or more, and also, it is preferably 5 or less, more preferably 3 or less, and even more preferably 2 or less. The hair to be treated with the agent for hair deforming treatment may be either the entire hair or a part thereof.

In order to increase interaction between the components (A) and hair proteins, and in order to promote a self-condensation reaction of the component (A) in hair to obtain the effects of the present invention, the heating temperature in the step (ii) is preferably 50°C or higher, more preferably 60°C or higher, and even more preferably 80°C or higher, and also, in order to suppress rapid evaporation of water during heating, the heating temperature is preferably 250°C or lower, more preferably 240°C or lower, and even more preferably 230°C or lower. Examples of the heating method include methods using a hair iron, an electric heating rod, a hot curler, etc.

The heating time applied in the step (ii) is selected, as appropriate, depending on the heating device and/or the heating temperature used. From the viewpoint of allowing the agent for hair deforming treatment to penetrate and/or diffuse into hair and to promote sufficient polymerization, the heating time is preferably 1 second or more, more preferably 1 minute or more, even more preferably 5 minutes or more, further preferably 15 minutes or more, and still further preferably 30 minutes or more, and also, in order to suppress hair damage, it is preferably 2 hours or less, more preferably 1 hour or less, and even more preferably 45 minutes or less.

Imparting a hair shape in the step (ii) includes both the imparting a straight shape and the imparting a curly shape. Examples of the method of giving a straight shape to hair include a method of blow-drying hair while pulling the hair with a tool such as a hand, a comb or a brush, and a method of heating hair using a hair iron. From the viewpoint of the ease of deformation, the method of using a hair iron is preferable. In order to impart a straight shape to hair, while heating the hair using a hair iron, a method of holding hair with a flat iron and then moving the flat iron from the roots to the tips, or a method of holding hair with a flat iron and then retaining it as is, while pulling the hair with a tool such as a hand, a comb or a brush, may be applied. Otherwise, a combination of the two above methods may also be applied. When a curly shape is imparted to hair, a method of curling hair with an electric heating rod, a hot curler, etc., and then retaining it as is, while heating the hair, a method of curling hair with a curl iron and then retaining it as is, and the like are applied.

The step (ii) is preferably carried out in an environment in which rapid evaporation of water is suppressed. Examples of a specific means for suppressing evaporation of water include a method of coating hair, to which the hair treatment agent has been applied, with a plastic film, a cap, etc., and a method of continuously spraying water vapor such as superheated vapor to hair.

A step of leaving hair, to which the hair treatment agent has been applied, may be inserted between the step (i) and the step (ii). In such a case, in order to allow the agent for hair deforming treatment to penetrate and/or diffuse into hair, the standing time is preferably 1 minute or more, more preferably 3 minutes or more, and even more preferably 5 minutes or more, and also, it is preferably 1 hour or less, more preferably 30 minutes or less, and even more preferably 20 minutes or less.

Moreover, from the viewpoint of promoting penetration of the agent for hair deforming treatment into hair, in the step of leaving hair, the heating may be performed. When the hair is heated, it is preferable to heat it at a temperature of from 40°C to 90°C.

Hair, to which the hair treatment agent has been applied, may be rinsed or may not be rinsed between the step (i) and the step (ii). From the viewpoint of sufficiently retaining the components of the hair treatment agent in hair, giving a semi-permanent shape to the hair after the treatment of hair, and further enhancing the effect of semi-permanently deforming the shape of the hair again by heating, it is preferable not to rinse the hair.

After completion of the step (ii), hair may be rinsed, or may not be rinsed. From the viewpoint of preventing a reduction in hair touch feeling due to redundant polymers, the hair is preferably rinsed.

By these treatments, the component (A) penetrates into hair, and thereafter, by heating the hair, an interaction of the component (A) with hair proteins occurs, and a protein structure is regulated. In addition, there is also a case where the component (A) is self-condensed in hair by heating the hair. This condensate is a thermoplastic condensate. For these reasons, the shape of hair can be deformed semi-permanently and thermoreversibly. Moreover, once the treatment is performed, hair can be freely and repeatedly deformed, semi-permanently or permanently, only by heating the hair, without applying the agent for hair deforming treatment again. Furthermore, the hair deformation imparted by the method of the present invention is not lost even by repeatedly washing the hair with shampoo.

### (Method for re-deforming treatment)

After hair has been subjected to deforming treatment by a method comprising the step (i) or the step (ii), the hair can be semi-permanently re-deformed to another shape by heating. When hair is re-deformed, heating imparted to the hair is preferably 30°C or higher, and more preferably 40°C or higher, and also, preferably 230°C or lower, more preferably 220°C or lower, and even more preferably 210°C or lower. In addition, when hair is subjected to re-deforming treatment, it is preferable not to apply any one of the agent for hair deforming treatment of the present invention and known agent for hair deforming treatments. Hereafter, specific procedures for carrying out a step of semi-permanently or permanently re-deforming hair to another shape by heating it will be described.

### • Case of re-deforming hair subjected to deforming treatment into curly shape into straight shape

In order to re-deform hair, which has been once subjected to deforming treatment into a curly shape, into a straight shape, a method of blow-drying hair with a dryer, while pulling the hair with a tool such as a hand, a comb or a brush, a method of heating hair with a hair iron, and the like are applied. From the viewpoint of the ease of deformation, the method of using the hair iron is preferable. In order to impart a straight shape to hair, while heating the hair with a hair iron, a method of holding hair with a hair iron and then moving the hair iron from the roots to the tips, or a method of holding hair with a hair iron and then retaining it as is, while pulling the hair with a tool such as a comb or a brush, may be applied. Otherwise, a combination of the two above methods may also be applied.

In this case, from the viewpoint of deforming the shape of hair semi-permanently or permanently, regardless of the type of a hair iron used, the material of a heating portion, a preset temperature, and the operational method of a hair iron, the attained temperature during the heating of hair (the temperature of hair) is preferably 120°C or higher, and more preferably 150°C or higher, and also, from the viewpoint of achieving both prevention of hair damage and semi-permanent or permanent deformation of the shape of hair, the attained temperature is preferably 230°C or lower, more preferably 220°C or lower, and even more preferably 210°C or lower. The temperature at which hair is heated can be measured, for example, using a radiation thermometer (Model No.: ST653) manufactured by SENTRY.

### • Case of re-deforming hair subjected to deforming treatment into straight shape into curly shape

In order to re-deform hair, which has been once subjected to deforming treatment into a straight shape, into a curly shape, a method of curling hair with a rod, a curler, etc., and then retaining it as is, while heating the hair, a method of curling hair with a hair iron and then retaining it as is, and the like are applied.

In this case, from the viewpoint of deforming the shape of hair semi-permanently or permanently, the attained temperature during the heating of hair (the temperature of hair) is preferably 30°C or higher, and more preferably 40°C or higher, and also, from the viewpoint of achieving both prevention of hair damage and semi-permanent or permanent deformation of the shape of hair, the attained temperature is preferably 180°C or lower, more preferably 120°C or lower, even more preferably 100°C or lower, further preferably 80°C or lower, and still further preferably 60°C or lower.

Even in a case where hair is re-deformed, upon heating it, either a method of heating hair, which is dried, or a method of heating hair after wetting it with water, may be applied. From the viewpoint of enhancing the effect of semi-permanently or permanently deforming the shape of hair, the method of heating hair after wetting it with water is preferable.

The heating time applied when hair is re-deformed is selected, as appropriate, depending on a heating tool used or a heating temperature. From the viewpoint of semi-permanently or permanently deforming the shape of hair, the heating time is preferably 1 second or more, more preferably 5 seconds or more, even more preferably 1 minute or more, further more preferably 5 minutes or more, still further preferably 15 minutes or more, and still further preferably 30 minutes or more. In addition, in order to suppress hair damage, it is preferably 2 hours or less, more preferably 1 hour or less, and even more preferably 45 minutes or less.

Particularly preferred embodiments of the above described method for treating hair of the present invention will be summarized below.
1) Hair is optionally wetted with water.
2) The hair treatment agent, which comprises the components (A) and (B), is applied to hair, and it is then allowed to penetrate into it.
3) The hair, to which the hair treatment agent has been applied, is optionally left for 1 minute or more and 1 hour or less. During this operation, the hair is optionally heated to a temperature of from 40°C to 90°C.
4) The hair is heated at a temperature of from 50°C to 250°C, to impart a shape to the hair.
5) The hair is optionally rinsed.
6) The hair is optionally heated at a temperature of from 40°C to 230°C to re-deform it.

Since the method for hair treatment of the present invention is a technique capable of freely changing hair based on a principle, which is completely different from a perm treatment using a reducing agent or a relaxer treatment using a strongly-alkaline hair treatment agent having pH 12 to 14, the present method does not comprise a step of applying a hair treatment agent comprising a reducing agent or a strongly-alkaline hair treatment agent having pH 12 to 14 to the hair. Accordingly, it can be said that, in comparison to the aforementioned conventional hair deformation methods, the method for hair treatment of the present invention is also advantageous in that hair can be deformed without being damaged.

With regard to the aforementioned embodiments, preferred aspects of the present invention will be further disclosed below.

### <1>

An agent for hair deforming treatment comprising the following components (A) and (B):
(A) a compound represented by the following formula (1), and
(B) water: 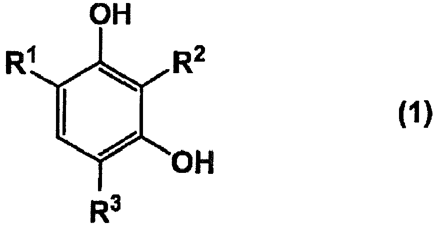
wherein R¹, R² and R³ each independently represent a hydrogen atom or a group represented by the following formula (2), wherein not all of R¹, R² and R³ simultaneously represent hydrogen atoms: 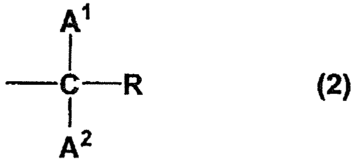 wherein R represents a hydrogen atom or a carboxy group, and A¹ and A² each independently represent a hydrogen atom, a hydroxyl group or a group represented by the following formula (3): 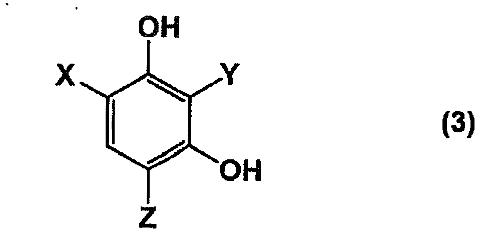 wherein any one of X, Y and Z represents a bond binding to the carbon atom shown in the formula (2), and the others represent a hydrogen atom or -CH(OH)COOH.

### <2>

The agent for hair deforming treatment according to <1> above, wherein the substituent represented by the formula (3) of the component (A) is preferably a group in which Y is a hydrogen atom, and either X or Z binds to the carbon atom shown in the formula (2) and the other is a hydrogen atom or -CH(OH)COOH.

### <3>

The agent for hair deforming treatment according to <1> or <2> above, wherein the component (A) is preferably one or two or more compounds selected from the group consisting of 2-(2,4-dihydroxypropyl)-2-hydroxyacetic acid, 2,2'-(4,6-dihydroxy-1,3-phenylene)bis(2-hydroxyacetic acid), 2,2-bis(2,4-dihydroxyphenyl)hydroxyacetic acid, 2-(5-(carboxy(2,4-dihydroxyphenyl)methyl)-2,4-dihydroxyphenyl)-2-hydroxyacetic acid, 2,2'-(4,6-dihydroxy-1,3-phenylene)bis(2-(2,4-dihydroxyphenyl)hydroxyacetic acid), 2-(5-(carboxy(2,4-dihydroxyphenyl)methyl)-2,4-dihydroxyphenyl)-2-(2,6-dihydroxyphenyl)acetic acid, 2-(5-(carboxy(2,4-dihydroxyphenyl)methyl)-2,4-dihydroxyphenyl)-2-(5-(carboxy(hydroxy)methyl)-2,4-dihydroxyphenyl)acetic acid, and 2,2'-(4,6-dihydroxy-1,3-phenylene)bis(2-(5-(carboxy(hydroxy)methyl)-2,4-dihydroxyphenyl)acetic acid).

### <4>

The agent for hair deforming treatment according to any one of <1> to <3> above, wherein the content of the component (A) is preferably 0.1 mass% or more, more preferably 0.5 mass% or more, even more preferably 1 mass% or more, and further preferably 1.5 mass% or more, and also, it is preferably 30 mass% or less, more preferably 25 mass% or less, even more preferably 23 mass% or less, further preferably 20 mass% or less.

### <5>

The agent for hair deforming treatment according to any one of <1> to <4> above, wherein the pH thereof is preferably 4 or less, more preferably 3 or less, even more preferably 2.5 or less, and further preferably 2 or less, and also, it is preferably 1 or more, more preferably 1.2 or more, and more preferably 1.5 or more.

### <6>

The agent for hair deforming treatment according to any one of <1> to <5> above, wherein, preferably, deformation of hair is not caused by the cleavage and recombination of the S-S bond of hair proteins.

### <7>

The agent for hair deforming treatment according to any one of <1> to <6> above, wherein the total amount of components which reduce proteins in hair is preferably less than 0.1 mass%, and more preferably less than 0.01 mass%, and even more preferably, the agent for hair deforming treatment does not comprise therein the components which reduce proteins in hair.

### <8>

The agent for hair deforming treatment according to <7> above, wherein the components which reduce proteins in hair are preferably selected from thiol, hydrogen sulfite, and salts thereof.

### <9>

The agent for hair deforming treatment according to <8> above, wherein the thiol is preferably thioglycolic acid, dithioglycolic acid, cysteine, acetylcysteine, or butyrolactonethiol.

### <10>

A method for hair treatment for semi-permanently or permanently deforming the shape of hair, which comprises the following steps (i) and (ii):
(i) a step of applying the agent for hair deforming treatment according to any one of <1> to <9> above to hair, and then allowing the agent to penetrate into the hair, and
(ii) a step of heating and shaping the hair into which the agent for hair deforming treatment has penetrated.

### <11>

The method for hair treatment according to <10> above, which preferably comprises a step of wetting hair before the step (i).

### <12>

The method for hair treatment according to <10> or <11> above, wherein the heating temperature in the step (ii) is preferably 50°C or higher, more preferably 60°C or higher, and even more preferably 80°C or higher, and also, it is preferably 250°C or lower, more preferably 240°C or lower, and even more preferably 230°C or lower.

### <13>

The method for hair treatment according to any one of <10> to <12> above, wherein the step (ii) is preferably carried out under an environment in which evaporation of water is suppressed.

### <14>

The method for hair treatment according to <13> above, wherein the environment in which evaporation of water is suppressed in the step (ii) is preferably provided by a method of coating hair, to which the hair treatment agent has been applied, with a plastic film or a cap, or a method of continuously spraying water vapor to hair.

### <15>

The method for hair treatment according to any one of <10> to <14> above, which preferably does not comprise a step of applying a hair treatment agent comprising a reducing agent or a strongly-alkaline hair treatment agent having pH 12 to 14 to the hair.

### <16>

The method for hair treatment according to any one of <10> to <15> above, wherein the mass of the agent for hair deforming treatment applied to hair in the step (i) is, at a bath ratio of the mass of the agent for hair deforming treatment to the mass of the hair (the mass of the agent for hair deforming treatment / the mass of the hair), preferably 0.05 or more, more preferably 0.1 or more, even more preferably 0.25 or more, and further preferably 0.5 or more, and also, it is preferably 5 or less, more preferably 3 or less, and even more preferably 2 or less.

### <17>

The method for hair treatment according to any one of <10> to <16> above, wherein the heating time in the step (ii) is preferably 1 second or more, more preferably 5 seconds or more, even more preferably 1 minute or more, further preferably 5 minutes or more, still further preferably 15 minutes or more, and still further preferably 30 minutes or more, and also, it is preferably 2 hours or less, more preferably 1 hour or less, and even more preferably 45 minutes or less.

### <18>

The method for hair treatment according to any one of <10> to <17> above, which preferably comprises a step of leaving hair, to which the hair treatment agent has been applied, between the step (i) and the step (ii).

### <19>

The method for hair treatment according to <18> above, wherein the leaving time is preferably 1 minute or more, more preferably 3 minutes or more, and even more preferably 5 minutes or more, and also, it is preferably 1 hour or less, more preferably 30 minutes or less, and even more preferably 20 minutes or less.

### <20>

The method for hair treatment according to <18> or <19> above, wherein hair is preferably heated at a temperature of from 40°C to 90°C when leaving hair.

### <21>

The method for hair treatment according to any one of <10> to <20> above, which preferably does not comprise a step of rinsing hair, to which the hair treatment agent has been applied, between the step (i) and the step (ii).

### <22>

The method for hair treatment according to any one of <10> to <21> above, which preferably comprises a step of rinsing hair after the step (ii).

### <23>

The method for hair treatment according to any one of <10> to <22> above, wherein hair is preferably re-deformed into a different shape by heating, after the step (ii).

### <24>

The method for hair treatment according to <23> above, wherein preferably the agent for hair deforming treatment is not applied, when hair is re-deformed.

### <25>

The method for hair treatment according to <23> or <24> above, wherein preferably the heating temperature when hair is re-deformed is preferably 30°C or higher, and more preferably 40°C or higher, and also it is preferably 230°C or lower, more preferably 220°C or lower, and even more preferably 210°C or lower.

### <26>

The method for hair treatment according to <10> to <25> above, which is a method for semi-permanently or permanently deforming the shape of hair.

### <27>

Use of the composition according to any one of <1> to <9> above for semi-permanent or permanent hair deformation.

### [Examples]

### Synthetic Example 1: Synthesis of mixture 1

5.0 g of Resorcin was dissolved in 21.0 g of water, and the mixed solution was then neutralized with 7.64 g of 48 mass% sodium hydroxide aqueous solution (this solution was defined as "solution a"). In addition, 1.39 g of glyoxylic acid monohydrate was dissolved in 24.3 g of water, and the mixed solution was then neutralized with 1.22 g of 48 mass% sodium hydroxide aqueous solution (this solution was defined as "solution b").

The solution a was mixed with the solution b, and the obtained solution was then heated to 80°C in a nitrogen atmosphere and was further heated for 45 minutes. After completion of the heating, the reaction mixture was cooled on ice, and then, 22.6 g of sulfuric acid was slowly added thereto, in order to adjust the pH of the solution to about 8.

Subsequently, the reaction solution was transferred into a separatory funnel, and was then washed with 50 mL of ethyl acetate twice. Thereafter, 3.7 g of sulfuric acid was added to the water layer, in order to adjust the pH to 1 or less.

Thereafter, extraction was carried out twice with 50 mL of ethyl acetate. The ethyl acetate layer was dehydrated with anhydrous sodium sulfate, and was then concentrated under a reduced pressure using an evaporator, while keeping the temperature at 40°C or lower. As a result, 2.6 g of a paste-like mixture 1 comprising 14.5 mass% ethyl acetate and 8.3 mass% water was obtained (wherein the content of the component (A) was 2.01 g).

¹H-NMR (400 MHz, DMSO-d6): δ11.8 ppm (carboxylic acid-COOH, calculated value: 1.4H (measured value: 1.1H)), 88.8-9.4 ppm (aromatic ring-OH, calculated value: 4.8H (measured value: 4.6H)), δ6.0-6.8 ppm (aromatic ring proton, calculated value: 6.8H (measured value: 6.8H)), δ5.0-5.2 (methine at the base of carboxylic acid, calculated value: 1.4H (measured value: 1.2H)). It is to be noted that the calculated value was calculated based on the results of the after-mentioned LC-MS.

The obtained product was analyzed by LC-MS. An area percentage method (calculating the ratio of the peak area of a product of interest to the total area of all of the detected peaks) was applied to components each having a molecular weight of 442 and 276, and the ratio of each component was calculated. The component having a molecular weight of 276 accounted for 58% of area ratio, and the component having a molecular weight of 442 accounted for 40% of area ratio.

As a result of the aforementioned analysis, it was found that the obtained component (A) was a mixture comprising the compounds having a molecular weight of 276 represented by the above structural formulae (iii) to (v) and the compounds having a molecular weight of 442 represented by the above structural formulae (viii) to (xii), at a ratio of 58 : 40.

Measurement by LC-MS was performed under the following conditions.

### <Analytic conditions and apparatuses for HPLC/UV/MS>

### HPLC/UV apparatuses and conditions

System: 1100 Series, manufactured by Agilent
AutoSampler: SIL-20A (Shimadzu Corporation)
Pump: G1312A (Agilent)
Column Oven: G1316A (Agilent)
UV: G1314A (Agilent)
Column: L-column ODS (4.6*150 mm) 5 µm
Column Temperature: 40°C
Eluent: A1: 0.1% TFA aqueous solution, B1: 0.1% TFA methanol
Gradient B: 10% (0-5 min) - 100% (15-20 min) - 10% (21-30 min)
Flow rate: 1.0 mL/min
Injected amount: 20 µL
UVD: 210 nm

### MS apparatus and conditions

MS: SL Series G1946D (Agilent)
Dry Gas Flow: 12 L/min
Nebulizer Pressure: 25 psi
Drying Gas Temp: 350°C
capillary Voltage: 4000°C
Ionization: ESI, positive
Fragmentor:90 V

### Examples 1 to 4 and Comparative Examples 1 to 2

The treatment agents shown in Table 1 were prepared, and the following three stages of hair treatment were then carried out using the agents. The shape-giving effect of each agent at each stage was evaluated. The results are also shown in Table 1. the pH value of each composition was measured by leaving the prepared composition at a room temperature (25°C) using a pH meter (manufactured by HORIBA / Model No.: F-52), without diluting each composition after the preparation thereof.

### <I: Imparting semi-permanent curly shape>

1. A Caucasian straight hair tress having 25 cm length and 0.5 g weight was wetted with tap water at 30°C for 30 seconds, and the wet tress was then wound around a plastic rod with a diameter of 14 mm, and was fixed with a clip.
2. 1 g of the treatment agent was applied to the tress wound around the rod, and the rod was then entirely covered with a plastic film for hermetical sealing. The tress was then heated for 1 hour in an oven, which was set at 90°C.
3. The tress was removed from the oven, and cooled to a room temperature.
4. The tress was removed from the rod, and was then rinsed with running tap water at 30°C for 30 seconds. Thereafter, a shampoo for evaluation was lathered on the tress for 60 seconds.
5. The tress was rinsed with running tap water at 30°C for 30 seconds, and was then immersed at an infinite bath ratio in tap water at 30°C for 60 seconds. Thereafter, the tress was gently pulled up out of the water while the root thereof being held, and water was then drained off by softly shaking it.
6. The tress was hung in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria)

A: The curl diameter is 1 time or more and less than 2 times as large as the diameter of the used rod (diameter: 14 mm) B: The curl diameter is 2 times or more and less than 3 times as large as the diameter of the used rod (diameter: 14 mm) C: The curl diameter is 3 times or more and less than 4 times as large as the diameter of the used rod (diameter: 14 mm) D: The curl diameter is 4 times or more and less than 50 times as large as the diameter of the used rod (diameter: 14 mm) E: The straight shape of hair tress is maintained, and the hair shape is not changed from before the treatment.

### <II: Imparting semi-permanent straight shape to semi-permanent curly shaped hair>

1. The tress, which had been evaluated in <I: Imparting semi-permanent curly shape> above, was combed for detangling, and a flat iron with an actual temperature of 180°C was slid on the tress at a rate of 5 cm/sec for six times.
2. The tress was rinsed with running tap water at 30°C for 30 seconds, and a shampoo for evaluation was lathered on the tress for 60 seconds. Thereafter, the tress was rinsed with running tap water at 30°C for 30 seconds, and was then towel dried.
3. The tress was dried with shaking so that the natural shape of hair could appear (wherein dryer was not used), and was then combed. Thereafter, the tress was hung, and was then visually observed.

### (Evaluation criteria)

A: The curl is not maintained, and the hair is completely deformed into straight hair.
B: The curl is weaker than before the treatment with a flat iron, however the hair is not completely deformed into straight hair.
C: The curly shape of hair tress is maintained, and the hair shape is not changed from before the treatment.

### <III: Imparting semi-permanent curly shape to semi-permanent straight shaped hair>

1. The tress, which had been evaluated in <II: Imparting semi-permanent straight shape to semi-permanent curly shaped hair> above, was wetted with tap water at 30°C for 30 seconds, and the wet tress was then wound around a plastic rod with a diameter of 14 mm, and was fixed with a clip.
2. The rod was entirely covered with a plastic film for hermetical sealing. Thereafter, the tress was heated for 1 hour in an oven, which was set at 40°C.
3. The tress was removed from the oven, and cooled to a room temperature.
4. The tress was removed from the rod, and was then rinsed with running tap water at 30°C for 30 seconds. Thereafter, a shampoo for evaluation was lathered on the tress for 60 seconds.
5. The tress was rinsed with running tap water at 30°C for 30 seconds, and was then immersed at an infinite bath ratio in tap water at 30°C for 60 seconds. Thereafter, the tress was gently pulled up out of the water while the root thereof being held, and water was then drained off by softly shaking it.
6. The tress was hung in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria)

A: The curl diameter is 1 time or more and less than 2 times as large as the diameter of the used rod (diameter: 14 mm) B: The curl diameter is 2 times or more and less than 3 times as large as the diameter of the used rod (diameter: 14 mm) C: The curl diameter is 3 times or more and less than 4 times as large as the diameter of the used rod (diameter: 14 mm) D: The curl diameter is 4 times or more and less than 50 times as large as the diameter of the used rod (diameter: 14 mm) E: The straight shape of hair tress is maintained, and the hair shape is not changed from before the treatment.

### <Formulation of shampoo for evaluation>

| Component | (mass%) |
|---|---|
| Sodium laureth sulfate | 15.5 |
| Lauramide DEA | 1.5 |
| Sodium benzoate | 0.5 |
| EDTA-2Na | 0.3 |
| Phosphoric acid | Amount for adjusting pH to 7 |
| Deionized water | balance |
| Total | 100 |

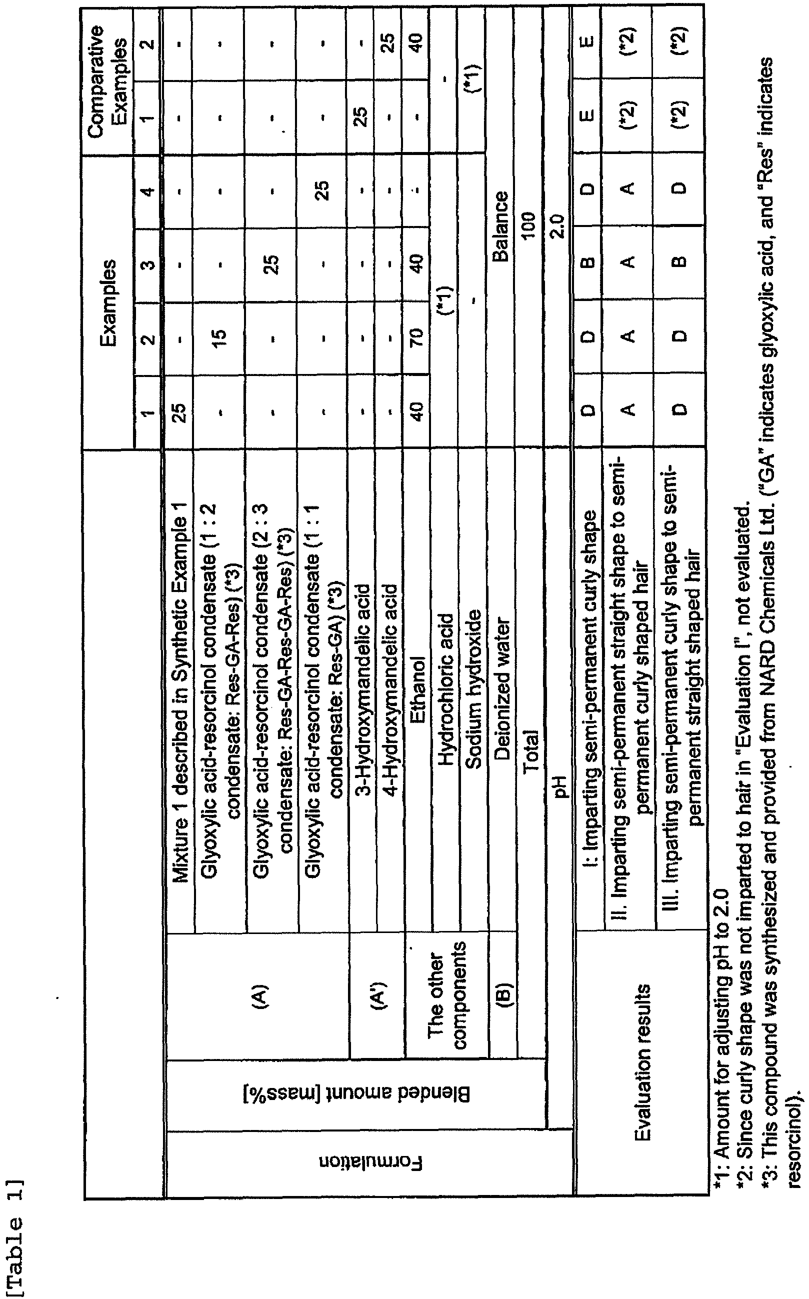

### Comparative Example 3

In accordance with Patent Literature 3 (US Patent No. 4278659), a hair treatment agent was prepared, and <I: Imparting semi-permanent curly shape> was then evaluated. Specifically, a hair treatment agent was prepared by the following procedures, and was then evaluated.

### <Preparation of hair treatment agent>

An aqueous solution having pH 2.0, which comprised 5 mass% glyceraldehyde and 5 mass% resorcinol, was prepared. This aqueous solution was heated to reflux for 1 hour to obtain a hair treatment agent.

### <I: Imparting semi-permanent curly shape>

1. A tress in length of 25 cm, which consisted of 0.5 g of Caucasian straight hair, was wetted with tap water at 30°C for 30 seconds, and the wet tress was then wound around a plastic rod with a diameter of 14 mm, and was fixed with a clip.
2. 1 g of the treatment agent prepared in <Preparation of hair treatment agent> above was applied to the tress, which was wound around the rod. The rod was entirely covered with a plastic film for hermetical sealing, and was then heated for 1 hour in an oven, which was set at 90°C.
3. The tress was removed from the oven, and cooled to a room temperature.
4. The tress was removed from the rod, and was then rinsed with running tap water at 30°C for 30 seconds. Thereafter, a shampoo for evaluation was lathered on the tress for 60 seconds.
5. The tress was rinsed with running tap water at 30°C for 30 seconds, and was then immersed at an infinite bath ratio in tap water at 30°C for 60 seconds. Thereafter, the tress was gently pulled up out of the water while the root thereof being held, and water was then drained off by softly shaking it.
6. The tress was hung in a laboratory for 2 hours, to be dried. The tress was combed, was then hung, and was then photographed from the side.

The hair treatment was carried out according to the above described procedures. After the hair had been treated with the hair treatment agent used in Comparative Example 3, the hair shape was the same as that of untreated hair, indicating that this hair treatment agent cannot impart a curly shape to the hair.

## Claims

1. An agent for hair deforming treatment comprising the following components (A) and (B):
(A) a compound represented by the following formula (1), and
(B) water: 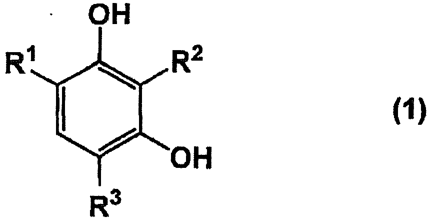 wherein R¹, R² and R³ each independently represent a hydrogen atom or a group represented by the following formula (2),
wherein not all of R¹, R² and R³ simultaneously represent hydrogen atoms: 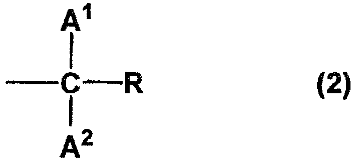 wherein R represents a hydrogen atom or a carboxy group, and A¹ and A² each independently represent a hydrogen atom, a hydroxyl group or a group represented by the following formula (3): 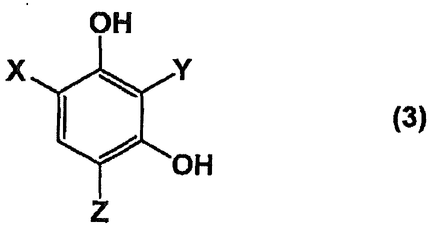 wherein any one of X, Y and Z represents a bond binding to the carbon atom shown in the formula (2), and the others represent a hydrogen atom or -CH(OH)COOH.

2. The agent for hair deforming treatment according to claim 1, wherein the content of the component (A) is 0.1 mass% or more and 30 mass% or less.

3. The agent for hair deforming treatment according to claim 1 or 2, wherein the pH thereof is 4 or less.

4. The agent for hair deforming treatment according to any one of claims 1 to 3, wherein the total amount of components which reduce proteins in hair is less than 0.1 mass%.

5. A method for hair treatment for semi-permanently or permanently deforming the shape of hair, which comprises the following steps (i) and (ii):
(i) a step of applying the agent for hair deforming treatment according to any one of claims 1 to 4 to hair, and then allowing the agent to penetrate into the hair, and
(ii) a step of heating and shaping the hair into which the agent for hair deforming treatment has penetrated.

6. The method for hair treatment according to claim 5, which comprises a step of wetting hair before the step (i).

7. The method for hair treatment according to claim 5 or 6, wherein the heating temperature in the step (ii) is 50°C or higher and 250°C or lower.

8. The method for hair treatment according to any one of claims 5 to 7, wherein the step (ii) is carried out under an environment in which evaporation of water is suppressed.

9. The method for hair treatment according to any one of claims 5 to 8, which does not comprise a step of applying a hair treatment agent comprising a reducing agent or a strongly-alkaline hair treatment agent having pH 12 to 14 to the hair.

10. The method for hair treatment according to any one of claims 5 to 9, wherein the hair is re-deformed by heating the hair after completion of the step (ii).
